# EUROPEAN PATENT APPLICATION

(11) **EP 3 385 391 A1**
(43) Date of publication of application: **10.10.2018**
(21) Application number: 16870742.0
(22) Date of filing: 30.11.2016
(51) Int. Cl.: C12Q 1/68, C12Q 1/06, G01N 33/48

(54) **METHOD FOR EVALUATING EFFICACY OF TREATMENT FOR MALIGNANT NEOPLASM BY MEASURING AMOUNT OF CTDNA**

(30) Priority: 30.11.2015 US 201562260973 P
(71) Applicant: DNA Chip Research Inc., Yokohama-shi, Kanagawa 230-0045 (JP); Osaka Prefectural Hospital Organization, Osaka 558-8558 (JP)
(72) Inventor: KATO, Kikuya, Osaka-shi Osaka 537-8511 (JP); IMAMURA, Fumio, Osaka-shi Osaka 537-8511 (JP); UCHIDA, Junji, Osaka-shi Osaka 537-8511 (JP); KUKITA, Yoji, Osaka-shi Osaka 537-8511 (JP); NISHINO, Kazumi, Osaka-shi Osaka 537-8511 (JP); KUMAGAI, Toru, Osaka-shi Osaka 537-8511 (JP)
(74) Representative: Dunleavy, Kevin James
(86) International application number: PCT/JP2016/085638
(87) International publication number: WO 2017/094805

(57) **Abstract**

The object of the invention is to provide a method for evaluating the efficacy of treatment for malignant neoplasm in a subject by measuring the amount of ctDNA in the subject. That is a method for evaluating the efficacy of treatment for malignant neoplasm in a subject, the method having (a) a step for measuring the amount of ctDNA in blood originating from the subject prior to the start of the treatment for malignant neoplasm, (b) a step for measuring the amount of ctDNA in blood originating from the subject after the start of the treatment for malignant neoplasm, and (c) a step for dividing the value obtained in step (b) by the value obtained in step (a).

## Description

### FIELD OF THE INVENTION

The present invention relates to a method for evaluating the efficacy of treatment for malignant neoplasm in a subject, and particularly to a method for evaluating the efficacy of treatment for malignant neoplasm by measuring the amount of ctDNA in a subject.

### BACKGROUND OF THE INVENTION

Conventionally, for diseases including cancer, brain tumor, hematopoietic cell malignant tumor such as leukemia and lymphoma, etc., treatments such as surgical treatment, chemotherapy, and radiotherapy have been applied, depending on the site the tumor exists and medical conditions such as progression.

In such a plurality of treatments, surgical treatment and radiotherapy are effective in topical treatment, while chemotherapy for cancer, for example, is effective as systemic treatment, because anticancer drugs are delivered into blood by intravenous injection or oral administration to suppress the division of cancer cells systemically and destroy them, and thereby therapeutic effect can be expected no matter where cancer cells exists in the body.

Since a large number of drugs have been developed for specific cancer and leukemia, chemotherapy has been used for treating lesions metastasized systemically, advanced cancer or the like by combining it with surgical treatment and radiotherapy.

In chemotherapy, although it is very effective at the beginning of treatment, some therapeutic drugs for specific cancer and leukemia lose their therapeutic effects by continuing the treatment, because malignant neoplasm gains resistance to those therapeutic drugs. By way of example, it is known that gefitinib (iressa), one of EGFR tyrosine kinase inhibitors (epidermal growth factor receptor tyrosine kinase inhibitors; EGFR-TKIs), shows anti-tumor effects at a high response rate (70-80%) for some patients with non-small cell lung cancer (Riely, G. J., Politi, K. A., and Miller, V. A., et al. (2006). Update on epidermal growth factor receptor mutations in non-small cell lung cancer. Clin. Cancer Res. 12, 7232-7241). However, resistance finally occurs to gefitinib in all patients to whom the drug was administered, which appears within about one year after the start of administration in most cases, and tumor increases again after the acquisition of the resistance (Sequist, L. V., Bell, D. W., Lynch, T. J., and Haber, D. A. (2007). Molecular predictors of response to epidermal growth factor receptor antagonists in non-small-cell lung cancer. J. Clin. Oncol. 25, 587-595).

The acquisition of resistance to therapeutic drugs, the efficacy of those therapeutic drugs and the like are now assessed by collecting a portion of a patient's tissue or organ and having a biopsy done; however, it is not preferable to use a biopsy because it is invasive and imposes significant burden on patients. It is also possible to confirm changes (increase or decrease) of tumor in target lesions by applying imaging diagnosis; however, in the case of imaging diagnosis, images are obtained after a lapse of several months (two months or more in most cases) after the administration of therapeutic drugs, because the permeation of therapeutic effects has to be waited, i.e., it takes a long time before assessing the efficacy of therapeutic drugs.

### SUMMARY OF THE INVNTION

The present invention was achieved in view of the abovementioned circumstances, and the object of the present invention is to provide a method for evaluating the efficacy of treatment for malignant neoplasm in a subject by measuring the amount of ctDNA in the subject.

The present inventors found that there were certain correlations between the amounts of tumor DNA in blood before and after treatment in patients and therapeutic effects thereof in some cases of non-small cell lung cancer or lung cancer and directed their attention to the possibility of evaluating therapeutic effects by measuring the amount of tumor DNA in blood.

As a result of conducting intensive studies in order to solve the abovementioned problems, the present inventors found that the efficacy of treatment for malignant neoplasm in patients could be evaluated by measuring the amounts of ctDNA before and after treatment in those patients and shedding light on the relationship between the ratios of those amounts and the response rates of therapeutic drugs.

More specifically, according to a first major viewpoint of the present invention, provided is a method for evaluating the efficacy of treatment for malignant neoplasm in a subject, the method comprising: (a) a step for measuring the amount of ctDNA in blood originating from the subject prior to the start of the treatment for malignant neoplasm; (b) a step for measuring the amount of ctDNA in blood originating from the subject after the start of the treatment for malignant neoplasm; and (c) a step for dividing the value obtained in step (b) by the value obtained in step (a).

In such a constitution, an objective method for evaluating clinical conditions (therapeutic effects) correlating to the ratio of amounts of ctDNA can be provided simply by measuring the amounts of ctDNA before and after a prescribed period of treatment to be evaluated. Furthermore, in such a constitution, the efficacy of treatment for malignant neoplasm can be evaluated in a subject non-invasively and conveniently, which can be conducive to selecting a therapeutic method suitable for the subject.

Furthermore, changes in ctDNA appear in blood flow relatively quickly after treatment; therefore, according to the method of the present invention, the efficacy of the treatment can be evaluated earlier than the image diagnosis of tumor, and information can be provided objectively and quickly in order to manage health or medical conditions of individual patients.

Moreover, according to one embodiment of the present invention, such a method can show that malignant neoplasm in the subject has shrunken when the value obtained in step (c) is 1/10 or less.

Moreover, according to another embodiment of the present invention, in the abovementioned first major viewpoint of the present invention, the subject is preferably a patient with non-small cell lung cancer or a patient with lung cancer.

Moreover, according to another embodiment of the present invention, in such a method, it is preferred that the amount of ctDNA be measured, in step (a), at any point in time within two months prior to the start of the treatment for the malignant neoplasm and that the amount of ctDNA be measured, in step (b), at any point in time within two months after the start of the treatment for the malignant neoplasm.

Moreover, according to another embodiment of the present invention, in such a method, it is preferred that the treatment for the malignant neoplasm be the administration of one or more drugs selected from cisplatin, pemetrexed, carboplatin, nab-paclitaxel, paclitaxel, bevacizumab, docetaxel, amrubicin, EGFR inhibitors, and chemotherapeutic agents, wherein it is preferred that the EGFR inhibitor be gefitinib or erlotinib.

Moreover, according to another embodiment of the present invention, in the abovementioned first major viewpoint of the present invention, the subject can be subjected to another treatment different from the treatment for the malignant neoplasm prior to step (a), wherein the abovementioned another treatment is surgical procedure, radiotherapy, or drug treatment. The drug treatment is preferably the administration of one or more drugs selected from cisplatin, pemetrexed, carboplatin, nab-paclitaxel, paclitaxel, bevacizumab, docetaxel, amrubicin, EGFR inhibitors, and chemotherapeutic agents.

According to a second major viewpoint of the present invention, a system for implementing the method according to the abovementioned first major viewpoint is provided. More specifically, provided is a therapeutic effect evaluation system for evaluating the efficacy of treatment for malignant neoplasm in a subject, the system comprising: (a) means for measuring the amount of ctDNA in blood originating from the subject prior to the start of the treatment for malignant neoplasm; (b) means for measuring the amount of ctDNA in blood originating from the subject after the start of the treatment for malignant neoplasm; and (c) means for dividing the value obtained in means (b) by the value obtained in means (a).

The features and marked actions and effects of the present invention other than those described above will be obvious to those skilled in the art by referring to the detailed description of the invention shown below and drawings.

### BRIEF DESCRIPTION OF THE DRAWINGS

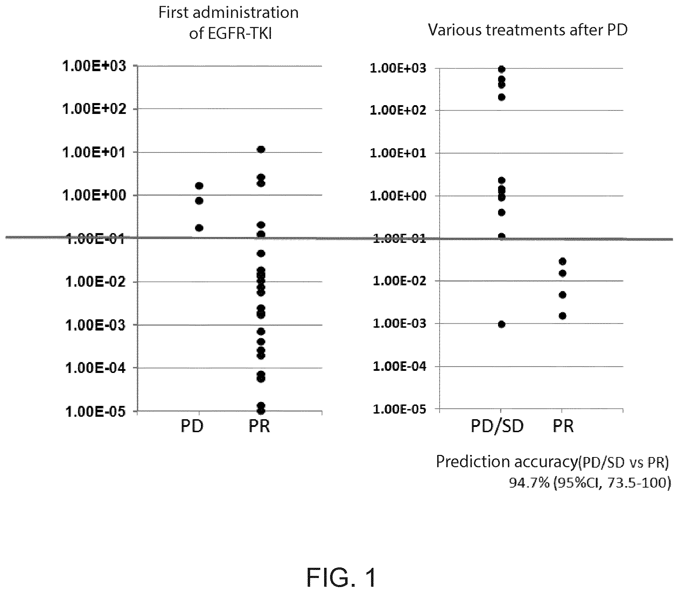

Fig. 1a is a graph formed by plotting MART for each of PD patients and PR patients after the first administration of EGFR-TKI according to one embodiment of the present invention. Fig. 1b is a graph formed by plotting MART for each of PD or SD patients and PR patients who received various treatments after the progression of disease caused by the acquisition of resistance to EGFR-TKI according to one embodiment of the present invention.

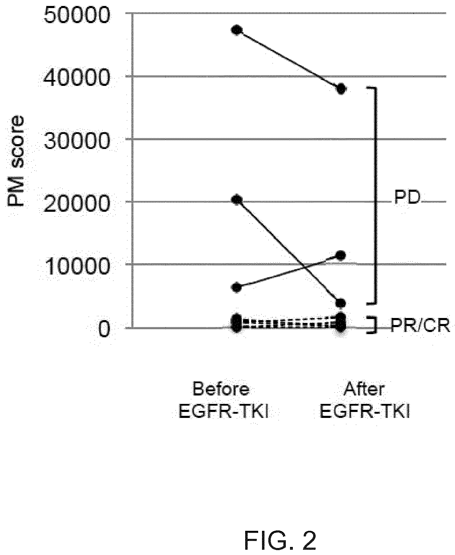

Fig. 2 is a graph showing changes in PM score before and after the first administration of EGFR-TKI according to one embodiment of the present invention.

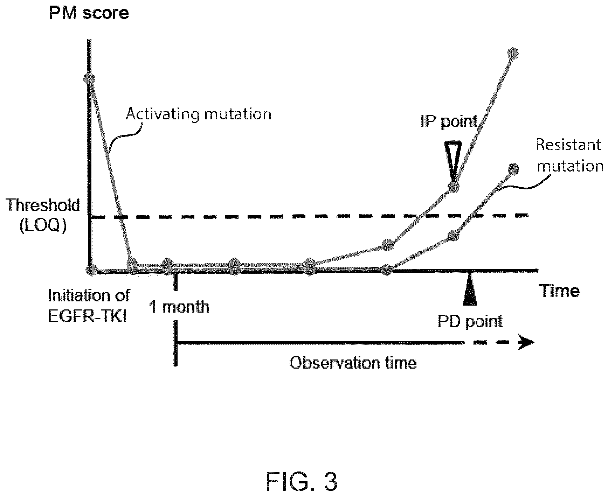

Fig. 3 is a schematic diagram showing changes in the amount of ctDNA in the EGFR-TKI treatment according to one embodiment of the present invention.

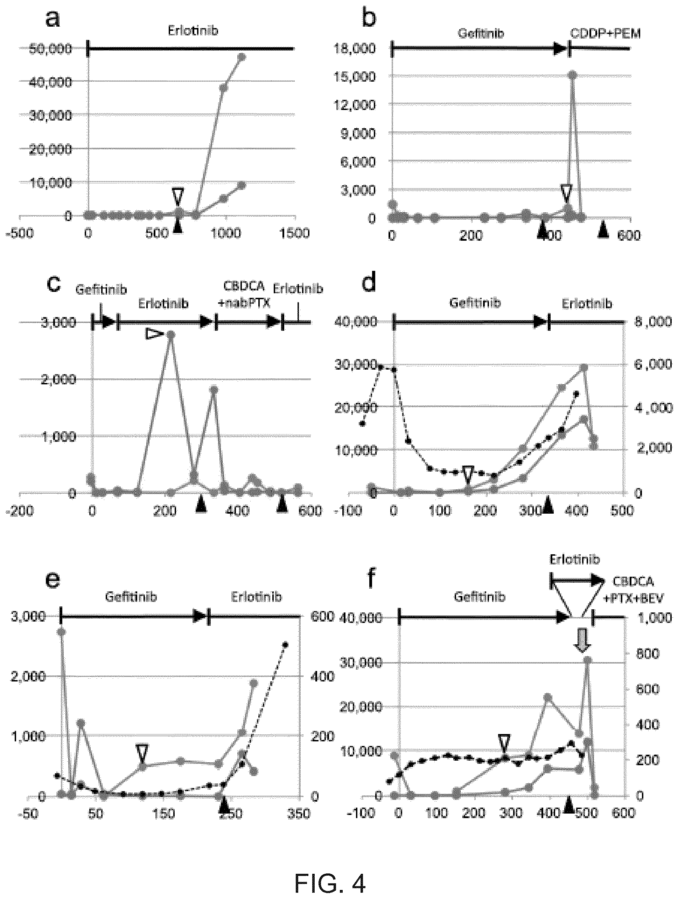

Fig. 4 is graphs showing changes in the amount of ctDNA for type I patients and type II patients according to one embodiment of the present invention. The vertical line shows the PM score of EGFR mutation (left) or the concentration of protein in CEA (a tumor marker) (µg/mL) (right). The horizontal line is the number of days after the start of administering EGFR-TKI. The horizontal line at the upper portion of each panel shows treatment, the perpendicular line shows the starting point of treatment, and the arrow head shows the ending point of treatment. The gray arrow under the horizontal line shows radiotherapy. The block arrow at the lower portion of each panel shows PD point. The white arrow of each panel shows IP point. Among data represented by solid lines, one having higher PM score is ctDNA having activating mutation and the other having lower score is ctDNA having resistant mutation. Only Fig. 4c shows one having higher PM score as ctDNA having resistant mutation and the other having lower score as ctDNA having activating mutation. If there is data represented by only one solid line, it shows ctDNA having activating mutation. The black broken line shows CEA. If there is no data exceeding detection threshold (PM score) or data is within the normal range (CEA), no data is shown for those patients. (a) Patient 1 (type I); (b) Patient 5 (type I); (c) Patient 11 (type I); (d) Patient 12 (type II); (e) Patient 13 (type II); and (f) Patient 14 (type II). The following describe abbreviations: CDDP (cisplatin); PEM (pemetrexed); CBDCA (carboplatin); nabPTX (nab-paclitaxel); PTX (paclitaxel); and BEV (bevacizumab).

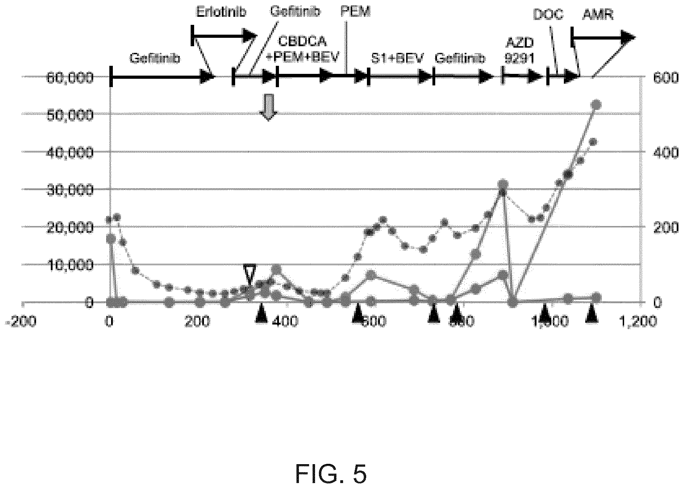

Fig. 5 is a schematic diagram showing changes in the amount of ctDNA in Patient 9 (type I) associated with a long-term observation period after the first progression of the disease according to one embodiment of the present invention. The following shows abbreviations: DOC (docetaxel); and AMR (amrubicin).

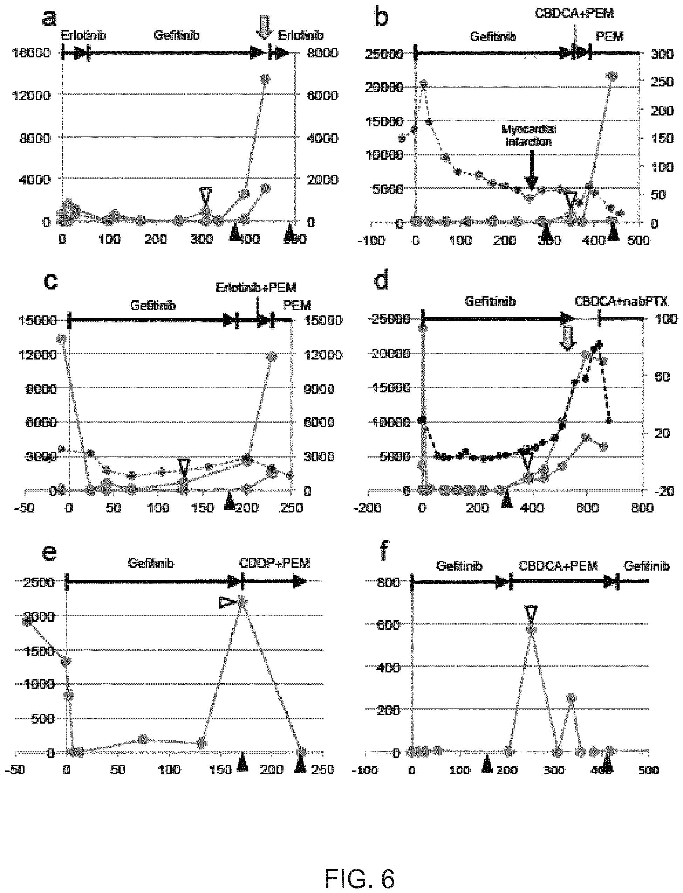

Fig. 6 is schematic diagrams showing changes in the amount of ctDNA in patients who are not shown in Fig. 4 and Fig. 5 according to one embodiment of the present invention. All the patients other than Patient 15 (type II) fall under the category of type I. The details of panels are the same as those in Fig. 4. (a) Patient 2; (b) Patient 3; (c) Patient 4; (d) Patient 6; (e) Patient 7; (f) Patient 8; (g) Patient 10; and (h) Patient 15.

### DETAILED DESCRIPTION OF THE INVENTION

The following describes one embodiment and examples according to the present invention with reference to drawings.

As described above, the present invention relates to a method for evaluating the efficacy of treatment for malignant neoplasm in a subject, the method comprising: (a) a step for measuring the amount of ctDNA in blood originating from the subject prior to the start of the treatment for malignant neoplasm; (b) a step for measuring the amount of ctDNA in blood originating from the subject after the start of the treatment for malignant neoplasm; and (c) a step for dividing the value obtained in step (b) by the value obtained in step (a).

As used herein, the term "malignant neoplasm" or "malignant tumor" refers to tumor and the like that can increase systemically by infiltrating into or metastasizing to other tissues. Although it generally refers to cancer, leukemia, etc., this term is not limited to those but can be used for any cell that is abnormally proliferated including epithelial dysplasia, adenoma, sarcoma, malignant lymphoma, osteosarcoma and myosarcoma.

Furthermore, as used herein, the evaluation of "the efficacy of treatment for malignant neoplasm" refers to the evaluation of efficacy of a given treatment or therapeutic drug when the treatment is given to a patient (a subject) having malignant neoplasm. By way of example, when the efficacy of an anticancer drug on tumor is assessed, the RECIST Guideline has been provided for objective tumor shrinkage effect at target lesions, wherein the efficacy of anticancer drugs are classified as follows: complete response (CP): disappearance of all target lesions; partial response (PR): at least a 30% decrease in the sum of the longest diameters of target lesions, taking as reference the baseline sum of longest diameters; progressive disease (PD): at least a 20% increase in the sum of the longest diameters of target lesions, taking as reference the smallest sum of longest diameters recorded since the treatment started; and stable disease (SD): neither sufficient shrinkage to qualify for PR nor sufficient increase to qualify for PD, taking as reference the smallest sum largest diameter since the treatment started. In a wide variety of diseases and treatments, the criteria of evaluating treatment for malignant neoplasm is not limited to one type; in the present invention, the evaluation of "the efficacy of treatment for malignant neoplasm" may be performed by a single effectiveness criterion, or the therapeutic effect may be evaluated by combining a plurality of effectiveness criteria.

Furthermore, as used herein, the term "ctDNA in blood originating from a subject" refers to free DNA originating from tumor, which suspends in blood of a subject, i.e., circulating tumor DNA (ctDNA). It is known that among DNA existing in blood only an extremely small amount of ctDNA generally exists as compared with normal cell DNA.

Furthermore, as used herein, the term "amount of ctDNA" refers to the value of quantified ctDNA described above and can be calculated as "plasma mutation (PM) score." The "PM score" can be defined as the number of activating mutations in population parameter reads (e.g., 100,000 reads) (including deletion mutation and substitution mutation; see also the description of activating mutation below). That is, the number of activating mutation in blood DNA can be defined as the "amount of ctDNA," because it is DNA originating from tumor in blood.

Furthermore, as used herein, the term "activating mutation" refers to mutations causing diseases and existing in target genes or target proteins on which molecular targeting drugs act or abnormal proteins that do not exist in normal cells. The term "resistant mutation" refers to mutations causing the acquisition of resistance after administering specific molecular targeting drugs.

Furthermore, the method according to the present invention makes it possible to evaluate the efficacy of treatment for malignant neoplasm in a subject by detecting activating mutations and resistant mutations in the subject, quantitatively measuring the amount of ctDNA, and then calculating MART.

More specifically, the amount of ctDNA during a predetermined period prior to treatment (a treatment method or a treatment drug) (A) used for assessing the efficacy and the amount of ctDNA during a predetermined period after the treatment (B) are measured, and then B is divided by A to find its ratio (B/A). As used herein, the ratio is referred to as "mutation allele ratio in therapy or MART." As the value obtained by the division decreases (e.g., 1/10 or less), the probability of PR or CR increases, and when it is 1/10 or more, the probability of PD increases.

The timing of measuring the amount of ctDNA prior to treatment used for assessing the efficacy may be any point in time within two months prior to the treatment, such as 50 days prior to the treatment, 40 days prior to the treatment, 35 days prior to the treatment, 30 days prior to the treatment, 28 days prior to the treatment, 21 days prior to the treatment, 20 days prior to the treatment, 15 days prior to the treatment, 14 days prior to the treatment, 10 days prior to the treatment, and 5 days prior to the treatment.

The timing of measuring the amount of ctDNA after treatment used for assessing the efficacy may be any point in time within two months after the treatment, such as 50 days after the treatment, 40 days after the treatment, 35 days after the treatment, 30 days after the treatment, 28 days after the treatment, 21 days after the treatment, 20 days after the treatment, 15 days after the treatment, 14 days after the treatment, 10 days after the treatment, and 5 days after the treatment. By making a measurement within two months after treatment, the therapeutic effect can be evaluated earlier than the therapeutic effect evaluated by imaging diagnosis.

The treatment for malignant neoplasm used for the assessment of efficacy may include the administration of one or more drugs selected from cisplatin, pemetrexed, carboplatin, nab-paclitaxel, paclitaxel, bevacizumab, docetaxel, amrubicin, EGFR inhibitors, and chemotherapeutic agents, wherein a patient for whom the amount of ctDNA was measured is subjected to the abovementioned treatment within two months of the measurement and subsequently within two months of the treatment the amount of ctDNA is measured again. The EGFR inhibitors are preferably gefitinib and erlotinib. Furthermore, according to one embodiment of the present invention, a subject may have already been under another treatment prior to the abovementioned treatments, i.e., a patient who was under certain treatment can be a subject, wherein the amount of ctDNA is measured in the subject, the abovementioned treatment is performed within two months of the measurement, and subsequently within two months of the treatment the amount of ctDNA is measured again.

According to one embodiment of the present invention, subjects for whom the efficacy of treatment for malignant neoplasm can be evaluated may include patients with non-small cell lung cancer and patients with lung cancer. The non-small cell lung cancer is a type of lung cancer, and it is known that epidermal growth factor receptor (EGFR) is involved therein in some cases. EGFR is a transmembrane glycoprotein having a molecular weight of about 170,000, and is known to be involved in the proliferation and continuation of cancer and expressed in various types of cancer including non-small cell lung cancer. While EGFR exists as a monomer in the inactive state, it forms a dimer when a growth factor such as EGF binds to a receptor and phosphorylates tyrosine residues in the intracellular domain of the receptor by making use of ATP at tyrosine kinase sites in the intracellular domain. As a result, proteins in the downstream of the signal transduction pathway is subjected to tyrosine phosphorylation in a chain reaction, and after a growth signal reaches the nucleus the cell cycle is advanced from G1 phase to S phase, resulting in the proliferation of cells. Moreover, the activation of EGFR tyrosine kinase increases the production of growth factors such as TGF-α and bFGF, stimulating the proliferation of cells through autocrine or paracrine signalling pathways, and also increases the production of vascular endothelial growth factor (VEGF), resulting in the acceleration of angiogenesis of cancer. It is believe that a series of these changes causes the grade of cancer to be increased, resulting in the progression of disease.

As described above, an EGFR inhibitor, preferably gefitinib or erlotinib may be administered to patients with non-small cell lung cancer as a treatment for malignant neoplasm used for assessing the efficacy. Gefitiniba has quinazoline as its basic framework, which is structurally similar to adenine of ATP and, therefore, reversibly binds to the ATP-binding site of intracellular tyrosine kinase site of EGFR, so that the binding of ATP is inhibited and activation suppressed.

It is known that gefitinib shows anticancer effects in some patients with non-small cell lung cancer, and it is also known that a greater part of those patients have mutations at a gene corresponding to the EGFR tyrosine kinase site. It is believed that this drug is effective because the abovementioned mutations change the structure of EGFR to one that can easily be bound by Getifinib while constantly keeping EGFR in the active state (activating mutation). Examples of the activating mutation include deletion mutations at exon 19 of the normal EGFR gene (ΔE746-A750, ΔE746-T751, ΔE746-A750 (ins RP), ΔE746-T751 (ins A/I), ΔE746-T751 (ins VA), ΔE746-S752 (ins A/V), ΔL747-E749 (A750P), ΔL747-A750 (ins P), ΔL747-T751, ΔL747-T751 (ins P/S), ΔL747-S752 (), ΔL747-752 (E746V), ΔL747-752 (E753S), ΔL747-S752 (ins Q), ΔL747-P753, ΔL747-P753 (ins S), ΔS752-1759, etc.), L858R point mutation, G719C, G719S, G719A, V689M, N700D, E709K/Q, S720P, V765A, T783A, N826S, A839T, K846R, L861Q, and G836D; however, all mutations can be included as long as those mutations can keep EGFR constantly in the active state. According to one embodiment of the present invention, the EGFR gene is used as a normal marker gene for non-small cell lung cancer.

It is also known that resistance occurs to patients administered with gefitinib and erlotinib. The resistance appears within about one year after the start of administration in many cases, and tumor increases again after the acquisition of the resistance. In about half cases, this is caused by the substitution of methionine for threonine that is the 790^{th} amino acid of the EGFR gene (resistant mutation, T790M point mutation). Crystal structure analysis shows the possibility that steric hindrance occurs as a result of the abovementioned change in amino acid, i.e., the substitution of methionine having a larger molecular weight, so that affinity to gefitinib and erlotinib is lowered. As one embodiment of the present invention, in addition to T790M, examples of such resistant mutations include D761Y, D770_N771 (ins NPG), D770_N771 (ins SVQ), D770_N771 (ins G), N771T, V769L, and S768I; however, any mutations can be included therein as long as those mutations cause the acquisition of resistance to gefitinib or erlotinib.

As one embodiment of the present invention, it is possible to evaluate the efficacy of treatment for malignant neoplasm in patients with non-small cell lung cancer by quantitatively measuring the amount of ctDNA through the detection of activating mutations and resistant mutations in the EGFR gene in those patients with non-small cell lung cancer, as described above, and then calculating MART.

As used herein, the term "marker gene" refers to a gene of cancer cells that has somatic cell mutations. The "marker gene" includes a gene targeted by a molecular targeting drug. More specifically, when a causative gene and a molecular targeting drug that can act on the causative gene are known in a specific disease, the marker gene refers to the causative gene targeted by the molecular targeting drug. As used herein, when a "marker gene" in a specific disease has somatic cell mutations, the term "normal marker gene" refers to a gene of normal cells that has no somatic cell mutation (normal gene). Furthermore, when one or more proteins become abnormal (e.g., abnormal proteins that do not exist in normal cells are produced) as a result of one or more genes (causative gene(s)) having somatic cell mutations in a specific disease, the normal gene refers to one or more genes of normal cells (normal genes) having no somatic cell mutation, which correspond to the one or more genes having the abovementioned mutation(s).

Furthermore, according to one embodiment of the present invention, the quantitative detection of activating mutations or resistant mutations, or the quantitative measurement of amount of ctDNA may be performed by a quantitative method such as the BEAMing technology. As used herein, BEAMing refers to a method for performing PCR reaction in an oil emulsion to immobilize PCR products originating from one molecule onto one nanoparticle, and then labelling and detecting normal and mutated bases at the site using different fluorescent dyes. In BEAMing and next-generation sequencers, DNA is amplified for each molecule using emulsion PCR; however, according to the method of the present invention, any quantitative analysis may be used as long as the analysis can distinguish normal and mutated bases from each other. By way of example, a wide variety of molecular biological methods can be used, including DNA chips, microarray methods, real-time PCR, a Northern blotting technique, a dot-blotting method, and quantitative reverse transcription-polymerase chain reaction (quantitative RT-PCR). In BEAMing, DNA is amplified for each molecule using emulsion PCR, and then each molecule is recovered using flow cytometry. Also, any type of next-generation sequencers can be used as long as it can determine base sequences in real time by synthesizing DNA using a DNA polymerase and one DNA molecule as a template and then detecting reaction for each base using fluorescence, light emission or the like, wherein any base recognition method, read length, reagent and the like that are used in next-generation sequencers may be used.

In order to evaluate the efficacy of treatment for malignant neoplasm as described above, it is preferred that the number of accumulated samples about the amount of ctDNA in patients be large, so that the evaluation method of the present invention can be enhanced in terms of accuracy. Furthermore, the present invention may be configured to store data about such accumulated samples in any database. In other words, the present invention can provide such a database for storing data and an analytical device or system for reading and implementing programs, etc. required for such data and comparative analysis. Such an analytical device or system can make it possible to accumulate data about the amounts of DNA before and after a predetermined period of treatment used for assessing the efficacy as well as information about actual therapeutic evaluation discovered by imaging diagnosis or the like (e.g., PD and PR) for each study subject, and retrieve and update accumulated data as required, so that the efficacy of treatment for malignant neoplasm can be evaluated for each subject at any time with ease.

Furthermore, such a system may be configured to connect a CPU incorporated in a computer system to an external memory device such as RAM, ROM, HDD and a magnetic disk as well as to an input/output interface (I/F) via a system bus. The input/output I/F is connected with an input device such as a keyboard and a mouse, an output device such as a display, and a communication device such as a modem. The external memory device is provided with a database for information about the amount of ctDNA, a database for information about imaging diagnosis, and a program storage part, each of which has a given memory area secured within the memory device.

Furthermore, such a system may have a system for measuring and analyzing the amount of ctDNA, wherein such a system for measuring and analyzing the amount of ctDNA may include a nucleic acid analyzer for analyzing nucleic acids on the basis of a biological sample containing nucleic acids that are collected from a subject using a blood collection tube for molecular diagnosis or the like as well as a MART analyzer for analyzing MART on the basis of analytical results, wherein those analyzers can be connected to each other via a communication network such as a dedicated line and a public line.

### EXAMPLES

The following describes the present invention in more detail using examples; however, the present invention is not intended to be limited by those examples.

### (Experimental techniques and materials)

The following describes experimental techniques and materials used in the present invention. Although the following experimental techniques are used in this embodiment, similar results can be achieved by using experimental techniques other than those described below.

### (Subjects)

Regardless of whether some types of treatment were given previously or not, patients with lung cancer having EGFR activating mutations were invited from November 2011 through March 2014 at Osaka Medical Center for Cancer and Cariovascular Diseases (University Hospital Medical Information Network Clinical Trials Registry UMIN000006764), and written consent was obtained from all the participants. The study relating to the present example was approved by the Ethics Committee of Osaka Medical Center for Cancer and Cariovascular Diseases, and its method was carried out in accordance with Approval Guidelines.

### (Blood sampling)

Blood samples for ctDNA assay were taken prior to the start of EGFR-TKI treatment as well as two weeks and four weeks after (14 days and 28 days after) the start of the treatment. To this schedule, +/- four extra days were given without counting holidays. After this period, blood was collected at intervals of two months as a target. Blood collection was continued at least until the objective progression of disease started or beyond this point in time as much as possible. Blood sampling ended on June 30, 2014.

### (Clinical evaluation of therapeutic response and disease progression)

Using RECIST version 1.1, therapeutic response was evaluated two months after the start of the first EGFR-TKI treatment. The progression of the disease during the EGFR-TKI treatment was also evaluated on the basis of RECIST criteria. Criteria recommended by the Guidelines for Diagnosis and Therapy of Lung Cancer (The Japan Lung Cancer Society) were also applied.

### (ctDNA assay)

In the assay system, mutations were searched by performing deep sequencing, i.e., by determining sequences of a large number of gene fragments. Exons 19, 20, and 21 of the EGFR gene were independently amplified by PCR from plasma DNA of patients, and deep sequencing was performed using Ion Torrent PGM (Thermo Fisher Scientific, Waltham, MA, USA). A diagnostic score referred to as plasma mutation (PM) score was defined as the number of reads having deletions (exon 19 deletion) or substitutions (exon 20, T790M; exon 21; L858R, L861Q) by reading 100,000 times. Parameters corresponding to limit of detection (LOD) and limit of quantification (LOQ) were determined, and thereby thresholds for detecting mutations were defined. Thresholds prior to the start of EGFR-TKI and up to two months after the start were set as LOQ for exon 19 deletion and LOD for L858R. In the case of evaluating the progression of the disease, detection threshold was set as LOQ (PM score = 300). According to multicenter studies conducted together with the present study, it was found that the possibility of false positive could be disregarded in the abovementioned setting, wherein estimated false-positive rates for exon 19 deletion, L858R, and T790M were 2%, 0%, and 1%, respectively.

Except that the present study used the most up-to-date reagents, the experimental procedure of the ctDNA assay was the same as one described in Kukita, Y. et al. Quantitative identification of mutant alleles derived from lung cancer in plasma cell-free DNA via anomaly detection using deep sequencing data. PLoS One 8, e81468, doi: 10.1371/journal.pone.0081468 (2013). The assay was conducted in order of sampling dates from November 2012 up to September 2014 at the rate of 12 to 24 samples per week.

### (Results)

### (Subjects and sample population)

A total of 52 people participated in the present study. The clinical characteristics of the patient population are shown in Table 1.

### [Table 1]

**Table 1. Clinical characteristics of patients**

| | All patients | Initial response | Disease progression |
|---|---|---|---|
| Total number of cases | 52 | 30 | 28 |

| Sex | | | |
|---|---|---|---|
| Male | 15 | 8 | 9 |
| Female | 37 | 22 | 19 |

| Age | | | |
|---|---|---|---|
| < 49 | 7 | 4 | 7 |
| 50-59 | 8 | 5 | 1 |
| 60-69 | 18 | 12 | 8 |
| 70-79 | 17 | 7 | 8 |
| 80-89 | 2 | 2 | 1 |

| Stage | | | |
|---|---|---|---|
| IIIA | 4 | 3 | 3 |
| IIIB | 7 | 5 | 2 |
| IV | 41 | 22 | 23 |

| Mutation in biopsy sample | | | |
|---|---|---|---|
| exon 19 deletion | 26 | 15 | 16 |
| L858R | 23 | 14 | 11 |
| double | 1 | 0 | 0 |
| 1.861Q | 1 | 1 | 1 |
| G719C | 1 | 0 | 0 |

| Treatment before EGFR-TKI | | | |
|---|---|---|---|
| none | 25 | 15 | 15 |
| surgery | 3 | 1 | 3 |
| surgery/radiation | 6 | 4 | 2 |
| surgery/chemotherapy | 3 | 1 | 3 |
| radiation | 7 | 5 | 2 |
| radiation/chemotherapy | 4 | 1 | 2 |
| chemotherapy | 4 | 3 | 1 |

| EGFR-TKI | | | |
|---|---|---|---|
| gefitinib | 41 | 22 | 24 |
| erlotinib | 11 | 8 | 1 |

| Effect of EGFR-TKI | | | |
|---|---|---|---|
| complete response (CR) | 2 | 1 | 1 |
| partial response (PR) | 30 | 23 | 22 |
| stable disease (SD) | 7 | 1 | 2 |
| progressive disease (PD) | 3 | 3 | 0 |
| not evaluable (NE) | 4 | 2 | 3 |

The patient information corresponds to the start of the EGFR-TKI treatment, and the total number of blood samples is 530. The PCR amplification of EGFR exon fragments was successful for all samples, and mutation data was obtained from all samples.

The present inventors examined whether or not the efficacy of the EGFR-TKI treatment could be evaluated using the dynamics of ctDNA. Since samples were lacking in 5 patients at the corresponding point in time, samples of 47 patients were used for analysis. Samples of 30 patients (63.8%) whose initial ctDNA levels exceeded the threshold level were used in the quantitative analysis shown below. The clinical characteristics of this patient subpopulation are shown in Table 1.

Mutation allele ratio in therapy (MART) was defined as the ratio between the PM score of activating mutations after the start of treatment and the PM score of activating mutations before the start of the treatment and was used as an indicator of therapeutic response. MART was calculated on the basis of the PM score two weeks or four weeks after the start of the treatment. Patients were classified into PD (progressive disease) cases and PR (partial response) cases; MART is plotted in the left panel of Fig. 1. MART exceeded 0.1 in all of three PD patients. On the other hand, MART exceeded 0.1 in 6 out of 24 PR cases (including one CR case). PM scores of these 9 cases are plotted in Fig. 2. The PM scores of PD patients were significantly higher than the PM scores of PR/CP patients.

### (Identification of cases suitable for long-term temporal analysis)

Next, the present inventors examined whether or not the start of progression of the disease can be assumed using the dynamics of ctDNA during the EGFR-TKI treatment. The target period was from one month after the start of the EGFR-TKI treatment until a point in time when the progression of the disease was detected. Only patients showing the objective progression of the disease (PD), which was caused by the acquisition of resistance during the EGFR-TKI treatment, were used for the present analysis. The EGFR-TKI treatment was cancelled for the following 11 patients before PD caused by the acquisition of resistance occurred: PD at the initial response (3 patients); deaths (3 patients); side-effects (4 patients); and patient preference (1 patient). PD did not occur in 6 patients during the observation period (median value: 358 days, minimum value: 198 days, maximum value: 747 days). PD occurred in the remaining 35 patients. In those cases, the following shows the statistics of periods until onset of PD: median value: 335 days; minimum value: 57 days, maximum value: 838 days.

In order to eliminate cases having insufficient data, cases having at least four samples within the range of 300 days including a point in time when PD occurred were selected, wherein 28 cases satisfied this criterion. The clinical characteristics of these cases are shown in Table 2, and summary statistics are shown in Table 1 (the number of samples: 351, and sampling intervals: median value: 43 days, bottom quartile: 22 days, and top quartile: 63 days).

### (Correlation between dynamics of ctDNA and start of disease progression)

Using retrospective datasets, the present inventors demonstrated that ctDNA levels of activating mutations and T790M mutations were suppressed until the start of the objective progression of the disease during the EGFR-TKI treatment only to increase subsequently (Uchida, J. et al. Dynamics of circulating tumor DNA represented by the activating and resistant mutations in the EGFR-TKI treatment. Cancer Sci. 2016, 107: 353-358). In order to make a comparison between the dynamics of ctDNA and the objective progression of the disease identified by imaging diagnosis, the present inventors defined initial positive (IP) point as the first point in time when the ctDNA level starts exceeding the threshold after the suppression. They identified the IP point for each patient and compared the value with the first point in time (PD point) when the objective progression of the disease was detected by imaging diagnosis. In order to facilitate the understanding of the IP point and the PD point, a schematic diagram of the dynamics of ctDNA is shown in Fig. 3. The IP point and the PD point are shown as time intervals (the number of days) from the date when the EGFR-TKI treatment was started (Table 2).

### [Table 2]

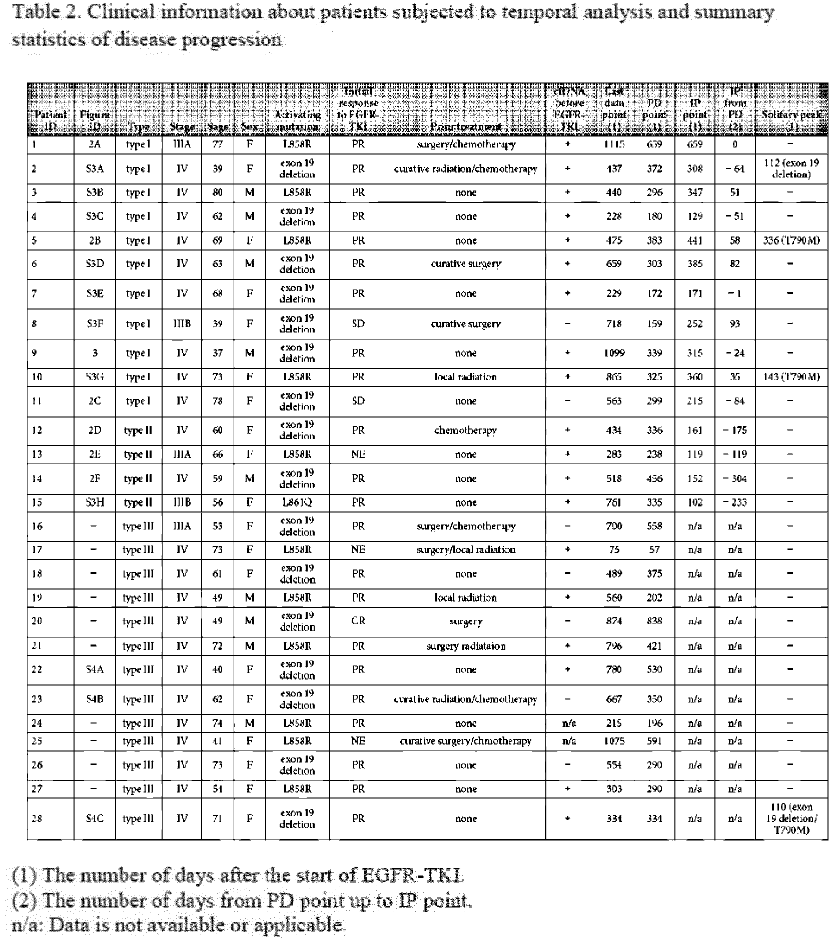

In "type I" patients (Patient 1 through Patient 11), time intervals between the IP point and the PD point were within the range of +/- 100 days (IP from PD; Table 2), and the number of data points therebetween was 1 or 0 (Fig. 4a through Fig. 4c, Fig. 5, and Fig. 6a through Fig. 6g). In consideration of time intervals between diagnostic tests, it is highly likely that the dynamics of ctDNA and imaging diagnosis show the same changes in medical conditions. In six patients, ctDNA levels declined at the time of the start of a new treatment (Fig. 4b through Fig. 4c, Fig. 5, and Fig. 6d). In Fig. 4 (exclusive of Fig. 4c), Fig. 5 and Fig. 6, among data represented by two solid lines, one having higher PM score is ctDNA having activating mutation and the other having lower score is ctDNA having resistant mutation. Only Fig. 4c shows one having higher PM score as ctDNA having resistant mutation and the other having lower score as ctDNA having activating mutation. If there is data represented by only one solid line, it shows ctDNA having activating mutation.

In Patient 12 through Patient 15 referred to as "type II" patients, the IP point preceded the PD point by 100 days or more (IP from PD; Table 2), and there was two data points therebetween (Fig. 4d through Fig. 4f and Fig. 6h). In those patients, the dynamics of ctDNA were different from imaging diagnosis and increased prior to the objective progression of the disease. While the dynamics of ctDNA of type I patients were uniform, those of type II patients were diversified. In Patient 12, the ctDNA level constantly increased after the IP point (Fig. 4d). In Patient 13, the ctDNA level of activating mutations first increased and maintained a constant level up to the PD point and then increased again as the ctDNA of T790M increased (Fig. 4e). In Patient 15, ctDNA did not increase in the objective progression of the disease, which most likely occurred because chemotherapeutic agents were used, though the fluctuation pattern was similar to that of Patient 13 (Fig. 6h). The dynamics of ctDNA of Patient 14 was complicated; one of the reasons is the intervention of other treatments (Fig. 4f).

Both activating mutations and T790M mutations increased after the IP point in eight patients (Fig. 4a, Fig. 4b, Fig. 4d through Fig. 4f, Fig. 5, Fig. 6a, Fig. 6c, and Fig. 6d). In those patients, the ctDNA level of T790M was always lower than the ctDNA level of activating mutations and increased in most cases. The present inventors directed their attention to the ctDNA of T790M of Patient 11 (Fig. 4c) and determined the IP point using the ctDNA of T790M only for this patient.

Patient 16 through Patient 28 did not show any PD-related increase and, therefore, were classified as "type III" (Table 2). The number of patients in type I, type II, and type III were 11.3 (39.3%), 4 (14.3%), and 13 (46.4%), respectively.

In all cases, carcinoembryonic antigen (CEA) levels were measured. The increase of CEA together with the objective progression of the disease was observed in 9 out of 28 patients (32.1%).

### (Response to various therapeutic methods)

Patient 9 received different treatments for a long period of time after the initial progression of the disease and is shown in Fig. 5 as an example. This patient was treated with various drugs, including gefitinib that was administered multiple times. Both the ctDNA of activating mutations and the ctDNA of T790M increased after the initial PD, which was immediately suppressed with therapeutic agents. Although the ctDNA of T790M was suppressed during the treatment with various therapeutic agents, activating mutations increased in parallel with the progression of the disease. When gefitinib was administered, the ctDNA of T790M increased, but when AZD9291, an EGFR-TKI targeting T790M, was administered, the ctDNA of T790M was suppressed. It is shown that the dynamics of T790M ctDNA indicate a cancer cell population associated with drug susceptibility different from that of the major cancer cell population. Other patients who have a long medical history of disease progression (i.e., Patient 10 and Patient 15) are shown in Fig. 6g and Fig. 6h.

### (Treatment other than EGFR-TKI)

Next, with regard to patients who had already been administered with EGFR-TKI and reached PD, the present inventors examined whether or not there was any correlation between the amounts of ctDNA before and after various treatments after reaching the PD point and the efficacy of those treatments. Changes in treatment after the initial progression of the disease are shown in Table 3. Those cases were classified on the basis of the RECIST criteria, except for cases receiving radiotherapy, and then plotted for MART (Fig. 1b). Data at a point in time close to the start of treatment were used for calculation. Related data is shown in Table 3.

### [Table 3]

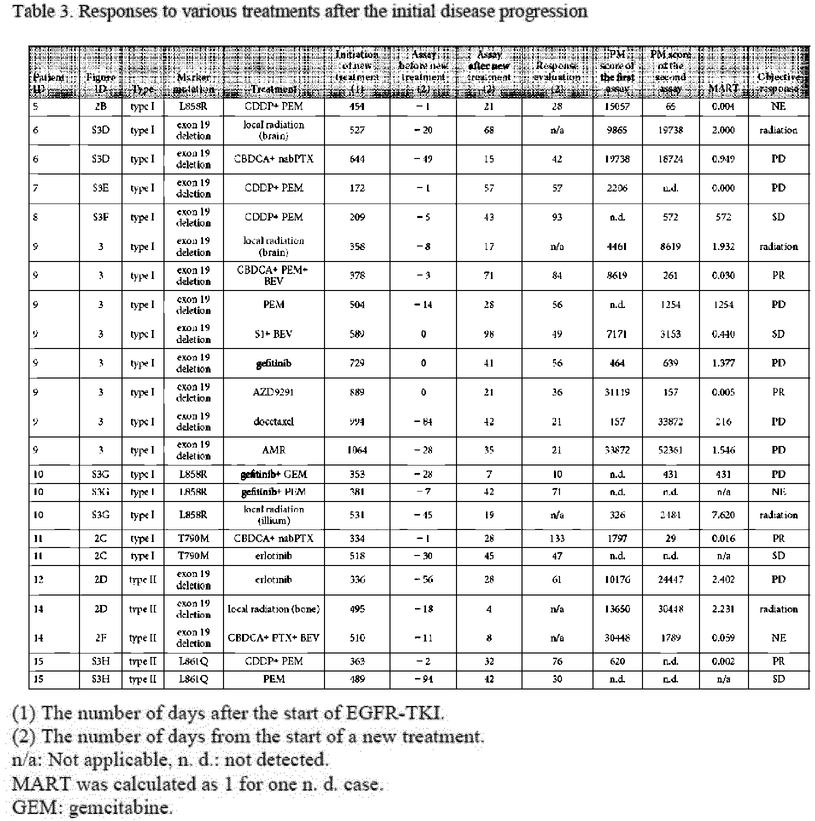

If the threshold (MART = 0.1) derived from the EGFR-TKI analysis is applied, accuracy was 94.7% (95% confidence interval, 73.5-100) for distinguishing PD/SD from PR. According to EGFR-TKI and therapeutic methods therefor, it is obvious that MART is a major parameter for evaluating therapeutic responses.

It goes without saying that the present invention is not limited to one embodiment described above but can be modified in various manners within the range of not changing the summary of the invention.

## Claims

1. A method for evaluating the efficacy of treatment for malignant neoplasm in a subject, the method comprising:
(a) a step for measuring the amount of ctDNA in blood originating from the subject prior to the start of the treatment for malignant neoplasm;
(b) a step for measuring the amount of ctDNA in blood originating from the subject after the start of the treatment for malignant neoplasm; and
(c) a step for dividing the value obtained in step (b) by the value obtained in step (a).

2. The method according to Claim 1, wherein when the value obtained in step (c) is 1/10 or less, it shows that malignant neoplasm in the subject has shrunken.

3. The method according to Claim 1, wherein the subject is a patient with non-small cell lung cancer or a patient with lung cancer.

4. The method according to Claim 1, wherein, in step (a), the amount of ctDNA is measured at any point in time within two months prior to the start of the treatment for the malignant neoplasm.

5. The method according to Claim 1, wherein, in step (b), the amount of ctDNA is measured at any point in time within two months after the start of the treatment for the malignant neoplasm.

6. The method according to Claim 1, wherein the treatment for the malignant neoplasm is the administration of one or more drugs selected from cisplatin, pemetrexed, carboplatin, nab-paclitaxel, paclitaxel, bevacizumab, docetaxel, amrubicin, EGFR inhibitors, and chemotherapeutic agents.

7. The method according to Claim 6, wherein the EGFR inhibitor is gefitinib or erlotinib.

8. The method according to Claim 1, wherein, prior to step (a), the subject was subjected to another treatment different from the treatment for the malignant neoplasm.

9. The method according to Claim 8, wherein the other treatment is surgical procedure, radiotherapy, or drug treatment, wherein the drug treatment is the administration of one or more drugs selected from cisplatin, pemetrexed, carboplatin, nab-paclitaxel, paclitaxel, bevacizumab, docetaxel, amrubicin, EGFR inhibitors, and chemotherapeutic agents.

10. A therapeutic effect evaluation system for evaluating the efficacy of treatment for malignant neoplasm in a subject, the system comprising:
(a) means for measuring the amount of ctDNA in blood originating from the subject prior to the start of the treatment for malignant neoplasm;
(b) means for measuring the amount of ctDNA in blood originating from the subject after the start of the treatment for malignant neoplasm; and
(c) means for dividing the value obtained in means (b) by the value obtained in means (a).
